# FASCICULE DE BREVET EUROPEEN

(11) **EP 1 615 014 B1**
(45) Date de publication et mention de la délivrance du brevet: **10.09.2008**
(21) Numéro de dépôt: 05291448.8
(22) Date de dépôt: 05.07.2005
(51) Int. Cl.: G01N 1/20

(54) **Dispositif de prélèvement d'échantillon liquide dans un circuit de pompage et procédé de nettoyage de ce dispositif**
Eine Vorrichtung zur Entnahme von flüssigen Proben und ein Reinigungsverfahren für die Vorrichtung
An apparatus for sampling liquid from a pumping circuit and a method for cleaning the apparatus

(30) Priorité: 06.07.2004 FR 0407484
(43) Date de publication de la demande: 11.01.2006
(73) Titulaire: Etablissements MAGYAR, 21000 Dijon (FR)
(72) Inventeur: Magyar, Georges, 21004 Dijon Cedex (FR)
(74) Mandataire: Bredema

(56) Documents cités:
- DE-A1- 3 627 849
- FR-A- 2 339 853
- US-A- 5 431 067

## Description

La présente invention concerne le domaine du prélèvement d'échantillon liquide dans un circuit en dépression. Il concerne plus particulièrement, mais non exclusivement, le domaine du prélèvement d'échantillon de lait dans un dispositif d'aspiration permettant de transférer le contenu d'un réservoir à lait dans une citerne de collecte.

Les producteurs de lait sont soumis à des exigences de qualité nécessitant une analyse du lait transféré d'un réservoir à lait du producteur vers une citerne de collecte.

Afin d'éviter que les opérations de prélèvement de lait ne soient trop coûteuses en termes de temps et de main d'oeuvre, il est intéressant que ces opérations soient effectuées automatiquement lors du pompage du lait dans le réservoir à lait du producteur.

On sait que les opérations de pompage du lait dans le réservoir à lait d'un producteur sont généralement réalisées au moyen d'une canne suceuse flexible raccordée à un circuit de pompage à dépression permettant l'aspiration du lait qui est ensuite conduit dans la citerne de collecte.

On connaît dans l'état de la technique un moyen de prélèvement d'échantillon liquide interposé dans le conduit d'aspiration du circuit de pompage. Ce moyen comprend une tête de prélèvement sur laquelle sont ménagés un orifice d'entrée et un orifice d'évacuation raccordés à des aiguilles creuses adaptées à traverser une membrane perforable d'un flacon d'échantillon pour délivrer dans ce dernier du liquide prélevé dans le conduit d'aspiration, le liquide excédentaire étant évacué par l'orifice d'évacuation.

Un tel dispositif a notamment comme inconvénient de ne pas permettre de contrôler le remplissage du flacon d'échantillon. Généralement, le flacon d'échantillon se trouve en fin de pompage rempli à ras bord et ce quelle que soit la position dans le flacon de l'extrémité de l'aiguille creuse raccordée à l'orifice d'évacuation. Outre les risques de débordement encourus, un tel flacon n'est pas facile à exploiter, eu égard notamment au fait que, dans le cas, par exemple, de l'analyse du lait, il faut prévoir dans le flacon un certain volume pour les réactifs d'analyse.

Le but de la présente invention est de proposer un moyen de prélèvement d'échantillon liquide amélioré permettant notamment de résoudre le problème évoqué ci-dessus.

L'invention concerne un dispositif de prélèvement d'échantillon liquide interposé dans le conduit d'un circuit de pompage, ledit dispositif comprenant une tête de prélèvement sur laquelle sont ménagés au moins un orifice d'entrée et un orifice d'évacuation raccordés par des circuits respectifs à des aiguilles creuses adaptées à traverser une membrane perforable d'un flacon d'échantillon.

Selon son acceptation la plus générale, elle est caractérisée en ce que ledit dispositif de prélèvement comprend un circuit de mise à l'air raccordé à une aiguille creuse adaptée à traverser ladite membrane perforable dudit flacon d'échantillon, l'extrémité de cette aiguille étant positionnée, à l'intérieur du flacon, au-dessus de l'extrémité de l'aiguille raccordée audit orifice d'évacuation qui détermine un niveau souhaité dudit échantillon liquide dans ledit flacon d'échantillon.

Selon un autre aspect de la présente invention, lesdites aiguilles raccordées aux orifices d'entrée et d'évacuation et ladite aiguille raccordée au circuit de mise à l'air sont portées par un même support commandé en translation verticale.

Selon un autre aspect de la présente invention, ladite tête de prélèvement est ménagée sur ledit support commandé en translation verticale.

Avantageusement, lesdits orifices d'entrée et de sortie sont ménagés au même niveau sur des faces respectivement amont et aval de ladite tête de prélèvement.

Selon un autre aspect de la présente invention, ledit support se déplace entre une position haute dans laquelle toutes lesdites aiguilles se trouvent au-dessus de ladite membrane et une position basse dans laquelle toutes lesdites aiguilles ont traversé ladite membrane, lesdits orifices d'entrée et d'évacuation restant dans tous les cas en communication avec ledit conduit dudit circuit de pompage.

Avantageusement, la position basse du support est réglable de manière à pouvoir régler le niveau souhaité de liquide dans le flacon d'échantillon.

Selon un autre aspect de la présente invention, un orifice, à travers lequel lesdites aiguilles peuvent traverser la paroi dudit conduit, est bouché par ladite membrane dudit flacon d'échantillon appliquée contre ledit orifice avec une certaine pression.

Avantageusement, ledit flacon est positionné sur un plateau porte-flacon commandé en translation verticale par un vérin.

Selon un autre aspect de la présente invention, lorsque ledit support est dans ladite position haute, les extrémités de toutes lesdites aiguilles se trouvent en communication avec ledit conduit dudit circuit de pompage.

Avantageusement, l'ouverture de l'aiguille raccordée au circuit de mise à l'air est tournée vers l'amont dudit conduit.

Ces deux dernières caractéristiques sont notamment avantageuses pour le nettoyage du dispositif.

L'invention concerne en effet également un procédé de nettoyage d'un dispositif de prélèvement d'échantillon liquide tel que défini précédemment. Ce procédé comprend une étape consistant à positionner ledit dispositif de manière à ce qu'aussi bien lesdits orifices d'entrée et d'évacuation que les extrémités de toutes lesdites aiguilles se trouvent en communication avec ledit conduit dudit circuit de pompage et une étape consistant à faire circuler dans ledit circuit de pompage un liquide de lavage sous pression importante.

La pression du liquide de lavage est telle que ce dernier va non seulement circuler dans les circuits d'entrée et d'évacuation mais va aussi pénétrer par l'extrémité de l'aiguille raccordée au circuit de mise à l'air et va circuler dans ce dernier pour jaillir par un orifice de mise à l'air. Le nettoyage de l'ensemble du dispositif de prélèvement d'échantillon liquide est ainsi assuré.

L'invention sera mieux comprise à la lecture des dessins annexés, correspondant à un mode de réalisation non limitatif, où :
- la figure 1 représente une vue schématique en coupe d'un dispositif de prélèvement d'échantillon selon l'invention, dans une première position de fonctionnement;
- la figure 2 représente une vue similaire à celle de la figure 1, représentant le même dispositif dans une seconde position de fonctionnement; et
- la figure 3 représente une vue similaire à celles des figures 1 et 2, représentant le même dispositif dans une position de nettoyage.

Le dispositif de prélèvement d'échantillon 1 selon l'exemple de réalisation décrit s'applique à un circuit de pompage de lait adapté au pompage du lait dans un réservoir à lait, par l'intermédiaire, par exemple, d'une canne suceuse flexible. Le dispositif 1 comprend un élément de conduit 10 adapté à être assemblé à d'autres éléments de conduit pour former un conduit d'aspiration de lait du circuit de pompage. Ainsi, le dispositif 1 peut être aisément intégré à un circuit de pompage quelconque.

Un dispositif de prélèvement d'échantillon 1 comprend globalement une tête de prélèvement 20 sur des faces amont et aval de laquelle sont ménagés un orifice d'entrée 21 et un orifice d'évacuation 22 raccordés à des aiguilles creuses respectives 31 et 32 montées sous ladite tête de prélèvement et s'étendant verticalement l'une à côté de l'autre vers le bas. Les orifices d'entrée 21 et d'évacuation 22 se trouvent au même niveau vertical sur la tête de prélèvement 20.

La tête de prélèvement 20 est montée à l'extrémité d'un bras vertical 15 commandé en translation verticale par un dispositif à air comprimé 18 assemblé sur une partie supérieure de l'élément de conduit 10.

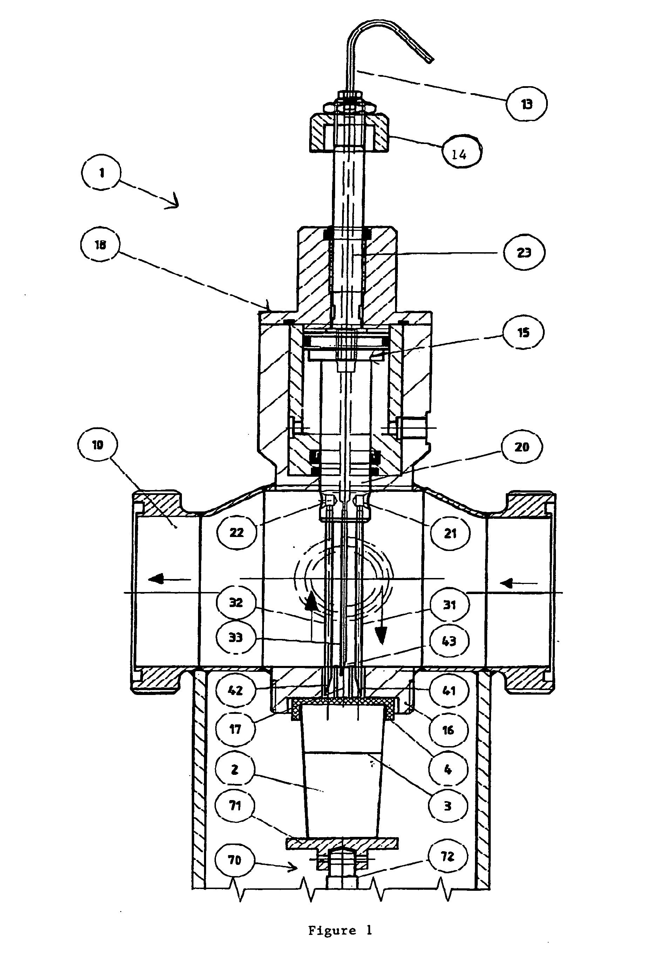

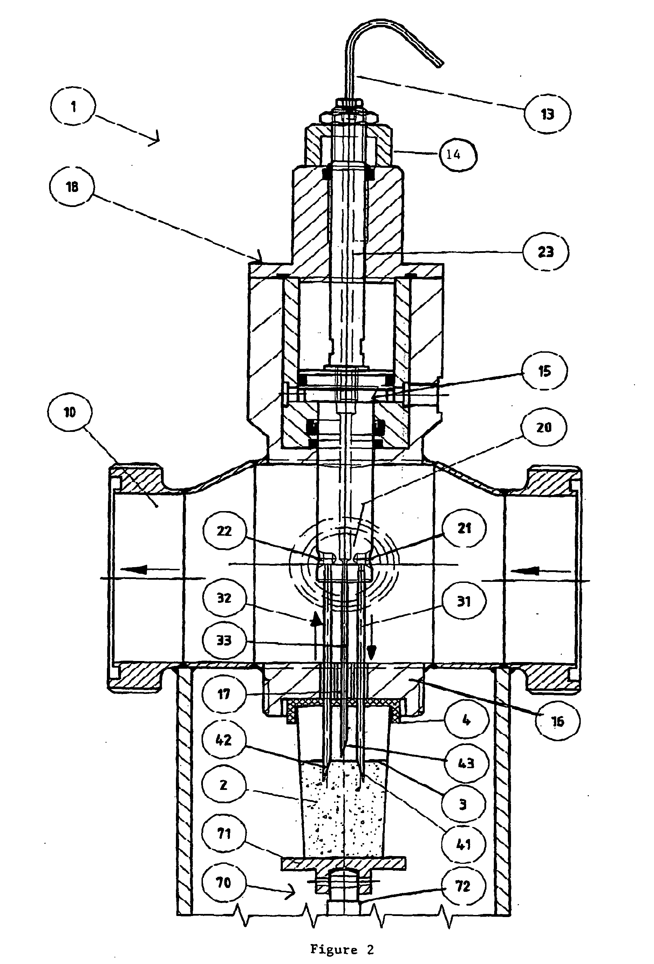

Le bras vertical 15 traverse la paroi supérieure de l'élément de conduit 10 par l'intermédiaire d'un moyen assurant l'étanchéité de l'élément de conduit au niveau de la liaison. Le bras vertical 15 évolue entre une position haute représentée sur la figure 1 et une position basse représentée sur la figure 2.

Une butée réglable 14 montée à l'extrémité supérieure du bras vertical 15 est adaptée à venir au contact d'une butée correspondante intervenant sur la partie supérieure du dispositif 18 en position basse du bras vertical 15. La position de la butée 14 sur l'arbre 15 est réglable, par exemple au moyen d'un dispositif à vis.

Dans la position haute, la tête de prélèvement 20 se trouve proche de la paroi supérieure de l'élément de conduit 10 tandis que dans la position basse, la tête de prélèvement 20 se trouve proche de l'axe de cet élément de conduit. Il convient de noter que dans les deux cas, les orifices d'entrée 21 et d'évacuation se trouvent en communication avec l'intérieur de l'élément de conduit 10.

Un conduit central 23 est ménagé le long de l'axe du bras vertical 15. Il s'étend sur toute la hauteur de ce bras entre, d'une part, un bec de mise à l'air 13 situé à l'extrémité supérieure du dispositif 1 et, d'autre part, une aiguille creuse 33 montée sous la tête de prélèvement 20 et s'étendant verticalement vers le bas entre les aiguilles 31 et 32.

Les extrémités inférieures 41 et 42 des aiguilles creuses 31 et 32 s'étendent au même niveau alors que l'extrémité inférieure 43 de l'aiguille creuse 33 s'étend au-dessus de ce niveau. Plus précisément, chacune des aiguilles 31, 32 et 33 étant ouverte sur toute son extrémité effilée, l'extrémité inférieure de la partie effilée 43 de l'aiguille 33 se trouve légèrement au-dessus des extrémités supérieures des parties effilées 41 et 42 des aiguilles 31 et 32. L'extrémité supérieure de la partie effilée 42 de l'aiguille 32 détermine le niveau souhaité d'un échantillon à l'intérieur d'un flacon, ainsi qu'on le voit bien sur la figure 2. Dans l'exemple représenté, l'extrémité supérieure de la partie effilée 41 de l'aiguille 31 s'étend au même niveau que l'extrémité supérieure de la partie effilée 42 de l'aiguille 32, c'est-à-dire au niveau souhaité de l'échantillon dans le flacon. L'extrémité inférieure de la partie effilée 43 de l'aiguille 33 se trouve, elle, juste au-dessus de ce niveau souhaité de l'échantillon dans le flacon.

Le niveau souhaité est réglable par l'intermédiaire de la butée 14 dont la position sur l'arbre 15 détermine la position basse de ce dernier.

Les ouvertures des aiguilles 42 et 43 sont tournées dans le même sens, opposé à l'ouverture de l'aiguille 41. Par rapport à l'élément de conduit 10, les aiguilles 42 et 43 sont ouvertes vers l'amont, comme l'orifice 21, alors que l'aiguille 41 est ouverte vers l'aval, comme l'orifice 22. De manière connue, ces aiguilles sont adaptées à traverser une membrane perforable d'un flacon d'échantillon pour que leurs extrémités effilées ouvertes se trouvent à l'intérieur du flacon, la membrane restant étanche.

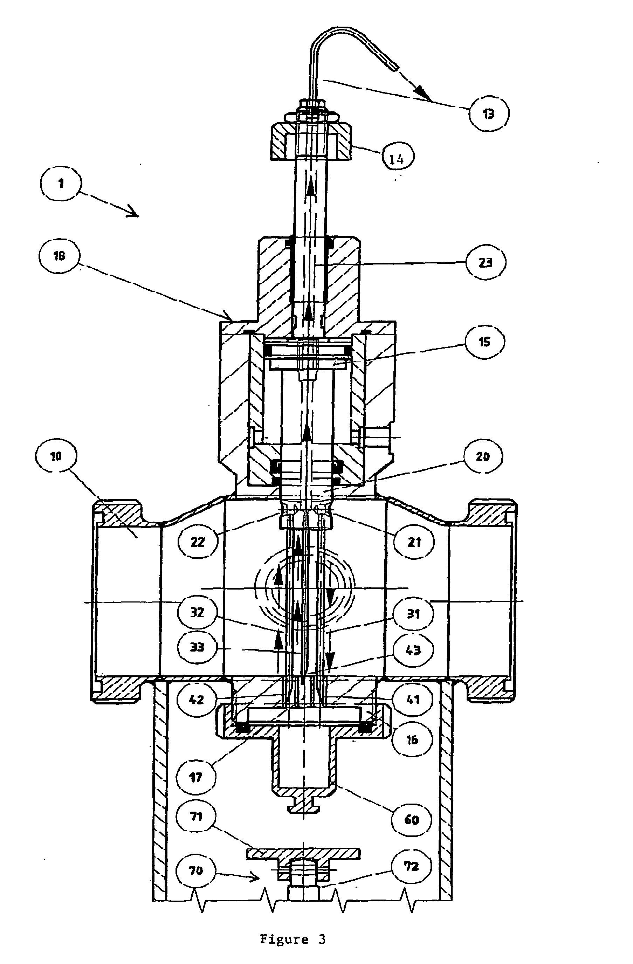

Les aiguilles 31, 32 et 33 peuvent traverser la paroi inférieure de l'élément de conduit 10 à travers un orifice. Cet orifice est délimité par un manchon 16 dont la partie inférieure définit un logement adapté à accueillir un flacon d'échantillon et dont la partie périphérique est adaptée à la fixation d'un bouchon 60, ainsi qu'on le voit sur la figure 3. Lorsqu'une membrane d'obturation de flacon d'échantillon est pressée contre l'orifice 17, ce dernier se trouve bouché de manière étanche. Lorsqu'il n'y a pas de flacon d'échantillon, par exemple lors d'une phase de nettoyage, on positionne le bouchon 60 sur le manchon 16.

Par ailleurs, un dispositif porte-flacon 70 est suspendu à une partie inférieure de l'élément de conduit 10 se trouvant en regard de la partie portant le dispositif à air comprimé 18. Ce dispositif porte-flacon 70 comprend un plateau porte-flacon 71 monté sur un vérin simple effet agissant sur un bras 72 coaxial au bras 15.

Le plateau 71 est adapté à recevoir un flacon 2 de dimension quelconque obturé par une membrane perforable 4. La hauteur du flacon importe peu puisque le vérin 72 permet de régler la hauteur pour que la membrane d'obturation 4 se trouve à la hauteur voulue, contre l'extrémité inférieure de l'orifice 17. Le diamètre de la membrane d'obturation doit être suffisant pour que cette dernière ferme complètement l'orifice 17. Le flacon est appliqué contre le manchon 16 avec une pression suffisante pour assurant l'étanchéité.

Une sonde (non représentée) permettant de détecter le débit de lait dans l'élément de conduit est installée dans ce dernier.

Un dispositif de commande automatique programmable (non représenté), connecté à la sonde, permet de commander les différents éléments décrits.

Une fois le flacon positionné et appliqué avec la pression de verrouillage voulue contre l'orifice 17, le dispositif de pompage est mis en service. La sonde de détection détecte l'arrivée du lait et le dispositif de commande enclenche une temporisation pendant laquelle la tête de prélèvement reste dans sa position haute. Cela permet d'assurer le rinçage sur circuit d'aspiration et le rinçage extérieur et intérieur de la tête de prélèvement, éliminant ainsi tout risque de contamination de l'échantillon par du lait provenant d'un fournisseur précédent.

Après la temporisation, le dispositif de commande actionne, par exemple au moyen d'une électrovanne, le dispositif 18 afin de faire descendre le bras 15 vers sa position basse. Les aiguilles 31, 32 et 33 vont perforer la membrane 4 et s'installer dans la position représentée sur la figure 2.

Le lait, entrant par l'orifice 21, est délivré dans le flacon 2 par l'intermédiaire de l'aiguille 31. Le lait excédentaire est évacué par l'aiguille 32 vers l'orifice 22. Le niveau 3 défini par l'extrémité supérieure de l'orifice 42 de l'aiguille 32 correspond au niveau souhaité de l'échantillon liquide dans le flacon d'échantillon. Une fois ce niveau atteint, il va rester constant, le lait excédentaire étant évacué par l'aiguille 32. La présence du circuit de mise à l'air constitué par l'aiguille 33 raccordée par le conduit 23 au bec de mise à l'air 13 va assurer que ce niveau n'est pas modifié par une aspiration de fin de pompage.

En référence à la figure 3, lorsque le bras 15 est dans la position haute, non seulement les orifices 21 et 22 mais aussi les extrémités 41, 42 et 43 des aiguilles 31, 32 et 33 se trouvent en communication avec l'intérieur du conduit 10. Ceci permet d'exécuter un nettoyage du dispositif 1 en même temps que le nettoyage du circuit de pompage. Le bras 15 se trouvant en position haute, l'on bouche l'orifice 17 à l'aide d'un bouchon 60 monté sur le manchon 16. Un liquide de nettoyage est ensuite injecté dans le circuit de pompage et traverse par conséquent l'élément de conduit 10. Le liquide de nettoyage, chaud et sous pression, entre par l'orifice 21, et sort par l'extrémité de l'aiguille 31. Par ailleurs, les ouvertures 42 et 43 des aiguilles 32 et 33 étant tournées vers l'amont du conduit, le liquide sous pression entre par ces ouvertures. Le liquide entré par l'ouverture 42 sort par l'orifice 22 tandis que le liquide entré par l'ouverture 43 remonte tout le conduit 23 et ressort par le bec de mise à l'air 13. Ainsi, tout le dispositif 1, y compris le circuit de mise à l'air, est nettoyé.

## Revendications

1. Dispositif de prélèvement d'échantillon liquide (1) interposé dans le conduit (10) d'un circuit de pompage, ledit dispositif comprenant une tête de prélèvement (20) sur laquelle sont ménagés au moins un orifice d'entrée (21) et un orifice d'évacuation (22) raccordés par des circuits respectifs à des aiguilles creuses (31, 32) adaptées à traverser une membrane perforable (4) d'un flacon d'échantillon (2), **caractérisé en ce que** ledit dispositif de prélèvement comprend un circuit de mise. à l'air (13, 23) raccordé à une aiguille creuse (33) adaptée à traverser ladite membrane perforable (4) dudit flacon d'échantillon (2), l'extrémité de cette aiguille (43) étant positionnée, à l'intérieur du flacon, au-dessus de l'extrémité (42) de l'aiguille (32) raccordée audit orifice d'évacuation qui détermine un niveau souhaité (3) dudit échantillon liquide dans ledit flacon d'échantillon.

2. Dispositif de prélèvement selon la revendication 1, **caractérisé en ce que** lesdites aiguilles (31, 32) raccordées aux orifices d'entrée (21) et d'évacuation (22) et ladite aiguille (33) raccordée au circuit de mise à l'air sont portées par un même support (15) commandé en translation verticale.

3. Dispositif de prélèvement selon la revendication 2, **caractérisé en ce que** ladite tête de prélèvement (20) est ménagée sur ledit support (15) commandé en translation verticale.

4. Dispositif selon l'une quelconque des revendications 2 et 3, **caractérisé en ce que** ledit support (15) se déplace entre une position haute dans laquelle toutes lesdites aiguilles (31, 32, 33) se trouvent au-dessus de ladite membrane (4) et une position basse dans laquelle toutes lesdites aiguilles ont traversées ladite membrane, lesdits orifices d'entrée (21) et d'évacuation (22) restant dans tous les cas en communication avec ledit conduit (10) dudit circuit de pompage.

5. Dispositif selon la revendication 4, **caractérisé en ce que** ladite position basse dudit support (15) est réglable.

6. Dispositif selon l'une quelconque des revendications précédentes, **caractérisé en ce que** lesdits orifices d'entrée (21) et d'évacuation(22) sont ménagés au même niveau sur des faces respectivement amont et aval de ladite tête de prélèvement (20).

7. Dispositif selon l'une quelconque des revendications précédentes, **caractérisé en ce qu'**un orifice (17) à travers lequel lesdites aiguilles (31, 32, 33) peuvent traverser la paroi dudit conduit (10) est bouché par ladite membrane (4) dudit flacon d'échantillon (2) appliquée contre ledit orifice avec une certaine pression.

8. Dispositif selon la revendication 7, **caractérisé en ce que** ledit flacon (2) est positionné sur un plateau (71) porte-flacon commandé en translation verticale par un vérin (72).

9. Dispositif selon l'une quelconque des revendications 4 à 8, **caractérisé en ce que**, lorsque ledit support (15) est dans ladite position haute, les extrémités de toutes lesdites aiguilles (31, 32, 33) se trouvent en communication avec ledit conduit (10) dudit circuit de pompage.

10. Dispositif selon l'une quelconque des revendications précédentes, **caractérisé en ce que** l'ouverture (43) de l'aiguille (33) raccordée au circuit de mise à l'air est tournée vers l'amont dudit conduit (10).

11. Procédé de nettoyage d'un dispositif de prélèvement d'échantillon liquide (1) selon l'une quelconque des revendications précédentes, **caractérisé en ce qu'**il comprend une étape consistant à positionner ledit dispositif de manière à ce que aussi bien lesdits orifices d'entrée (21) et d'évacuation(22) que les extrémités (41, 42, 43) de toutes lesdites aiguilles (31, 32, 33) se trouvent en communication avec ledit conduit (10) dudit circuit de pompage et une étape consistant à faire circuler dans ledit circuit de pompage un liquide de lavage sous pression.

## Claims

1. A device for taking a liquid sample (1) interposed in the duct (10) of a pumping system, said device comprising a sampling head (20) whereon are arranged at least one inlet (21) and one outlet (22) connected by respective systems to hollow needles (31, 32) adapted for going through a punctable membrane (4) of a sample bottle (2), **characterized in that** said sampling device includes a venting system (13, 23), connected to a hollow needle (33) adapted for going though said punctable membrane (4) of said sample bottle (2), with the end of such needle (43) being positioned, inside the bottle, above the end (42) of the needle (32) connected to said outlet which determines a desired level (3) of said liquid sample in said sample bottle.

2. A sampling device according to claim 1, **characterized in that** said needles (31, 32) connected to the inlets (21) and outlets (22) and said needle (33) connected to the venting system are held by the same vertical translation controlled support (15).

3. A sampling device according to claim 2, **characterized in that** said sampling head (20) is arranged on said vertical translation controlled support (15).

4. A device according to any one of claims 2 and 3, **characterized in that** said support (15) is moved between an upper position where all of said needles (31, 32, 33) are above said membrane (4) and a lower position where all of said needles have gone through said membrane, with said inlets (21) and outlets (22) remaining, in any case, in communication with said duct (10) of said pumping system.

5. A device according to claim 4, **characterized in that** said lower position of said support (15) can be adjusted.

6. A device according to any one of the preceding claims, **characterized in that** said inlets (21) and outlets (22) are arranged at the same level, on respectively backward and frontward faces of said sampling head (20).

7. A device according to any one of the preceding claims, **characterized in that** a hole (17) through which said needles (31, 32, 33) can go through the wall of said duct (10) is closed by said membrane (4) of said sample bottle (2) applied against said hole, with some pressure.

8. A device according to claim 7, **characterized in that** said bottle (2) is positioned on a bottle-holding tray (71) the vertical translation of which is controlled by an actuator (72).

9. A device according to any one of claims 4 to 8, **characterized in that**, when said support (15) is in the upper position, the ends of all of said needles (31, 32, 33) are communicating with said duct (10) of said pumping system.

10. A device according to any one of the preceding claims, **characterized in that** the opening (43) of the needle (33) connected to the venting system is turned backward of said duct (10).

11. A method for cleaning a device for taking a liquid sample (1) according to any one of the preceding claims, **characterized in that** it includes a step of positioning said device so that said inlets (21) and outlets (22) as well as the ends (41, 42, 43) of all of said needles (31, 32, 33) are communicating with said duct (10) of said pumping system and a step of circulating a pressurized washing liquid in said pumping system.

## Patentansprüche

1. Vorrichtung für die Entnahme von flüssigen Proben (1), die in der Leitung (10) eines Pumpkreislaufs zwischengebaut ist, wobei die besagte Vorrichtung einen Entnahmekopf (20) umfaßt, auf dem mindestens eine Eintrittsöffnung (21) und eine Entleerungsöffnung (22) gestaltet sind, die durch jeweilige Kreisläufe mit Hohlnadeln (31, 32) verbunden sind, die geeignet sind, eine perforierbare Membran (4) eines Probenfläschchens (2) zu durchbrechen, **dadurch gekennzeichnet, daß** die besagte Vorrichtung für die Entnahme einen Entlüftungskreis (13, 23) umfaßt, der mit einer Hohlnadel (33) verbunden ist, die geeignet ist, die besagte perforierbare Membran (4) des besagten Probenfläschchens (2) zu durchbrechen, wobei das Ende dieser Nadel (43) im Innern des Fläschchens über dem Ende (42) der Nadel (32) positioniert ist, die mit der besagten Entleerungsöffnung verbunden ist, die einen gewünschten Füllstand (3) der besagten flüssigen Probe im besagten Probenfläschchens bestimmt.

2. Entnahmevorrichtung nach Anspruch 1, **dadurch gekennzeichnet, daß** die besagten mit der Eintrittsöffnung (21) und der Entleerungsöffnung (22) verbundenen Nadeln (31, 32) und die besagte mit dem Entlüftungskreislauf verbundene Nadel (33) von einem gleichen Träger (15) getragen werden, der in vertikaler Translation gesteuert wird.

3. Entnahmevorrichtung nach Anspruch 2, **dadurch gekennzeichnet, daß** der besagte Entnahmekopf (20) auf dem besagten Träger (15) gestaltet ist, der in vertikaler Translation gesteuert wird.

4. Vorrichtung nach einem beliebigen der vorstehenden Ansprüche 2 und 3, **dadurch gekennzeichnet, daß** sich der besagte Träger (15) zwischen einer oberen Position, in der sich alle besagten Nadeln (31, 32, 33) über der besagten Membran (4) befinden, und einer unteren Position, in der alle besagten Nadeln die besagte Membran durchbrochen haben, bewegt, wobei die besagte Eintrittsöffnung (21) und Entleerungsöffnung (22) in allen Fällen in Verbindung mit der besagten Leitung (10) des besagten Pumpkreislaufs bleiben.

5. Vorrichtung nach Anspruch 4, **dadurch gekennzeichnet, daß** die besagte untere Position des besagten Trägers (15) regelbar ist.

6. Vorrichtung nach einem beliebigen der vorstehenden Ansprüche, **dadurch gekennzeichnet, daß** die besagte Eintrittsöffnung (21) und Entleerungsöffnung (22) auf gleicher Höhe auf der oberen beziehungsweise unteren Seite des besagten Entnahmekopfes (20) gestaltet sind.

7. Vorrichtung nach einem beliebigen der vorstehenden Ansprüche, **dadurch gekennzeichnet, daß** eine Öffnung (17), durch die die besagten Nadeln (31, 32, 33) die Wand der besagten Leitung (10) durchqueren können, durch die besagte Membran (4) des besagten Probefläschchens (2) verschlossen ist, die mit einem gewissen Druck an die besagte Öffnung angedrückt ist.

8. Vorrichtung nach Anspruch 7, **dadurch gekennzeichnet, daß** das besagte Fläschchen (2) auf einer Tragplatte (71) für Fläschchen positioniert ist, die durch einen Zylinder (72) in vertikaler Translation gesteuert wird.

9. Vorrichtung nach einem beliebigen der vorstehenden Ansprüche 4 bis 8, **dadurch gekennzeichnet, daß**, wenn der besagte Träger (15) in der besagten oberen Position ist, die Enden aller besagten Nadeln (31, 32, 33) sich in Verbindung mit der besagten Leitung (10) des besagten Pumpkreislaufs befinden.

10. Vorrichtung nach einem beliebigen der vorstehenden Ansprüche, **dadurch gekennzeichnet, daß** die Öffnung (43) der an den Entlüftungskreis angeschlossenen Nadel (33) nach oben von der besagten Leitung (10) gedreht ist.

11. Verfahren für die Reinigung einer Vorrichtung für die Entnahme von flüssigen Proben (1) nach einem beliebigen der vorstehenden Ansprüche, **dadurch gekennzeichnet, daß** es einen Schritt umfaßt, der darin besteht, die besagte Vorrichtung so zu positionieren, daß sowohl die besagte Eintrittsöffnung (21) und Entleerungsöffnung (22), als auch die Enden (41, 42, 43) aller besagten Nadeln (31, 32, 33) sich in Verbindung mit der besagten Leitung (10) des besagten Pumpkreislaufs befinden, und einen Schritt, der darin besteht, im besagten Pumpkreislauf eine Waschflüssigkeit unter Druck zirkulieren zu lassen.
